# EUROPEAN PATENT SPECIFICATION

(11) **EP 1 654 983 B1**
(45) Date of publication and mention of the grant of the patent: **13.08.2008**
(21) Application number: 05024139.7
(22) Date of filing: 04.11.2005
(51) Int. Cl.: A61B 5/07, A61B 5/00

(54) **Apparatus and method for receiving, selecting and combining signals**
Vorrichtung und Verfahren zum Empfangen, zum Auswählen und zum Kombinieren von Signalen
Système et procédé pour la réception, la sélection et la combination des signaux

(30) Priority: 04.11.2004 US 624756 P
(43) Date of publication of application: 10.05.2006
(60) Divisional application: 08160079.3
(73) Proprietor: Given Imaging Ltd., 20692 Yoqneam (IL)
(72) Inventor: Bettesh, Ido, Haifa 34788 (IL); Nisani, Micha, Ramot Itzhak Nesher 36781 (IL)
(74) Representative: HOFFMANN EITLE

(56) References cited:
- EP-A- 0 667 115
- WO-A-20/04036803
- US-A1- 2002 165 592
- US-B1- 6 334 073

## Description

### FIELD OF THE INVENTION

The present invention relates generally to an in-vivo sensing system. In particular, it is related to an apparatus and method for processing signals communicated between an in-vivo transmitting device and a plurality of receiving devices to obtain, for example, captured image signals and/or other telemetry data.

### BACKGROUND OF THE INVENTION

In an in-vivo sensing system, an in-vivo device, for example, an ingestible device that may move through the gastrointestinal (GI) tract, and that may collect data and transmit the data to a receiver system are known in the art. The in-vivo device, for example, a capsule of a cylindrical shape, may have a wireless telemetry system allowing transmission of desired collected data continuously or as a burst at pre-programmed time intervals via a miniature antenna via radio frequency (RF). The radio transmission is then received by, for example, a small receiver attached to the patient or in a clinic.

Because of the constraint of physical dimensions imposed on the sensing device, (for example, in one embodiment, the sensing device has to be able to move through the GI tract), and the general desire to have a small sensing device that may be swallowed by or otherwise inserted into a patient with minimal discomfort, the size of an antenna that is used inside the sensing device may be consequently limited. The dimensions of the antenna may actually be much smaller than the wavelength of a radio frequency at which the antenna operates. For example, the size of antenna may be in the order of one percentage or less of the wavelength. Because antenna efficiency, measured by the amount of RF power radiated, is proportional to its area, the small physical size may cause a significant decrease in the antenna efficiency, which affects overall communication channel power budget. On the other hand, during transmission of the radio frequency signal from the sensing device inside a patient's body to a receiver/recorder outside, the quality of radio frequency signal may suffer degradation due to power attenuation by the human body. In addition, potential noise sources in a surrounding environment where in-vivo diagnostics may be conducted may also contribute to degradation in signals detected at the receiver/recorder.

EP-A-06 667 115 describes an in-vivo video camera system including a swallowable capsule, a transmitter and a reception system. The swallowable capsule includes a camera system and an optical system for imaging an area of interest on to the camera system. The transmitter transmits the video output of the camera system and the reception system receives the transmitted video output. The reception system may comprise an antenna array having a multiplicity of antennas. Furthermore, a signal sampler can be provided that comprises a multiplicity of receivers, one for each antenna. Furthermore, a data processor is provided for processing the received data.

WO 2004/036803 A describes a system for transfer of a signal, such as an energizing signal, to a transmitting in-vivo sensing device. The in-vivo device includes a signal transmitter and a signal receiver unit. Furthermore, an external phased array antenna is provided. The phased array antenna receives a signal from the sensing device and then transmits a signal to the device phased in the reverse order to that of receipt of the signal from the sensing device.

### SUMMARY OF THE INVENTION

The subject-matter of the invention is defined in the appended claims.

It is an objective of this invention to provide an in-vivo communication system comprising an in-vivo sensing device comprising a transmitting device and a reception system comprising a plurality of receiving devices; a signal selector or multiplexer connected to said plurality of receiving devices; and a signal processing device connected to said signal multiplexer,

The sensing device is able to communicate with the reception system through the transmitting device and the plurality of receiving devices, and the signal selector is able to select two or more signals from a plurality of received signals provided by the plurality of receiving devices, and output the two or more signals to the signal processing device.

It is a further objective of this invention that the signal selector measures a relative signal strength of the plurality of received signals and selects the two or more signals based upon an order of said relative signal strength.

It is a further objective of this invention that the signal processing device is able to adjust said selected two or more signals to be substantially in-phase.

It is a further objective of this invention that the signal processing device is able to combine the phase adjusted two or more signals substantially in-phase.

It is a further objective of this invention that the signal processing device is able to adjust the phase adjusted two or more signals to have substantially same amplitudes.

It is a further objective of this invention that the signal processing device is able to combine the phase and amplitude adjusted two or more signals substantially in-phase and in substantially same amplitudes.

It is a further objective of this invention that the in-vivo sensing device is a swallowable capsule.

It is a further objective of this invention that the transmitted signal comprises an image or video signal.

It is a further objective of this invention that the transmitting device includes a transmitting antenna.

It is a further objective of this invention that the plurality of receiving devices comprises two or more receiving antennas.

It is an objective of this invention to provide a method of reproducing a transmitted signal from a transmitting device that comprises receiving a plurality of signals at a plurality of receiving devices; selecting two or more signals from the plurality of received signals; and constructing the transmitted signal from the selected two or more signals.

It is a further objective of this invention that the method of constructing comprises detecting phases of the selected two or more signals; and adjusting the phases of the selected two or more signals to be substantially in-phase.

It is a further objective of this invention that the method further comprises combining the phase-adjusted two or more signals to reproduce the transmitted signal.

It is a further objective of this invention that the method further comprises measuring amplitudes of the phase adjusted two or more signals; and adjusting the amplitudes of the phase adjusted two or more signals to have substantially same amplitudes.

It is a further objective of this invention that the method further comprises combining the phase and amplitude adjusted two or more signals to reproduce the transmitted signal.

It is a further objective of this invention that the method of selecting comprises selecting two or more signals from the plurality of received signals by an order of relative signal strength.

### BRIEF DESCRIPTION OF THE DRAWINGS

Embodiments of the invention are illustrated by way of example and not limitation in the figures of the accompanying drawings, in which like reference numerals indicate corresponding, analogous or similar elements, and in which:
Fig. 1 is a conceptual illustration of an exemplary in-vivo sensing system, which contains a sensing device, a receiver/recorder, and a workstation, in accordance with some embodiments of the invention;
Fig. 2 is a simplified block-diagram illustration of an exemplary in-vivo sensing system, in accordance with some embodiments of the invention;
Fig. 3 is a simplified schematic illustration of a set of antennas, including one antenna, in an in-vivo sensing device, in accordance with some embodiments of the invention;
Fig. 4 is a simplified schematic block-diagram illustration of a receiver circuitry for selection of a signal from a set of receiving antennas, in accordance with one embodiment of the invention;
Fig. 5 is a simplified schematic block-diagram illustration of a receiver circuitry for combining two or more signals from a set of receiving antennas, in accordance with another embodiment of the invention;
Fig. 6 is a simplified block diagram illustration of a method for combining two or more received signals to reproduce a signal transmitted by an in-vivo sensing device; and
Figs. 7A and 7B are simplified schematic block diagram illustration and schematic physical illustration, respectively, of a part of a front-end receiver in accordance with another embodiment of the invention.

It will be appreciated that for simplicity and clarity of illustration, elements shown in the figures have not necessarily been drawn to scale. For example, the dimensions of some of the elements may be exaggerated relative to other elements for clarity.

### DETAILED DESCRIPTION OF THE INVENTION

In the following detailed description, numerous specific details are set forth in order to provide a thorough understanding of embodiments of the invention. However it will be understood by those of ordinary skill in the art that the embodiments of the invention may be practiced without these specific details. In other instances, well-known methods, procedures, components and circuits have not been described in detail so as not to obscure the embodiments of the invention.

Some embodiments of the present invention are directed to a typically swallowable device that may passively or actively progress through the gastro-intestinal (GI) tract, pushed along, in one example, by natural peristalsis. Other embodiments are directed at in vivo sensing devices that may be passed through other body lumens such as through blood vessels, the reproductive tract, etc. The device may be a sensing device, an imager, a diagnostic device, a therapeutic device, or a combination thereof. According to one embodiment, the device may include an image sensor. Devices and methods, including in-vivo sensing devices, receiving systems, and display systems, according to embodiments of the present invention may be similar to embodiments described in International Application publication number WO 01/65995, and/or in US Patent Number 5,604,531, each of which are assigned to the common assignee of the present invention. Devices as described herein may have other configurations and sets of components.

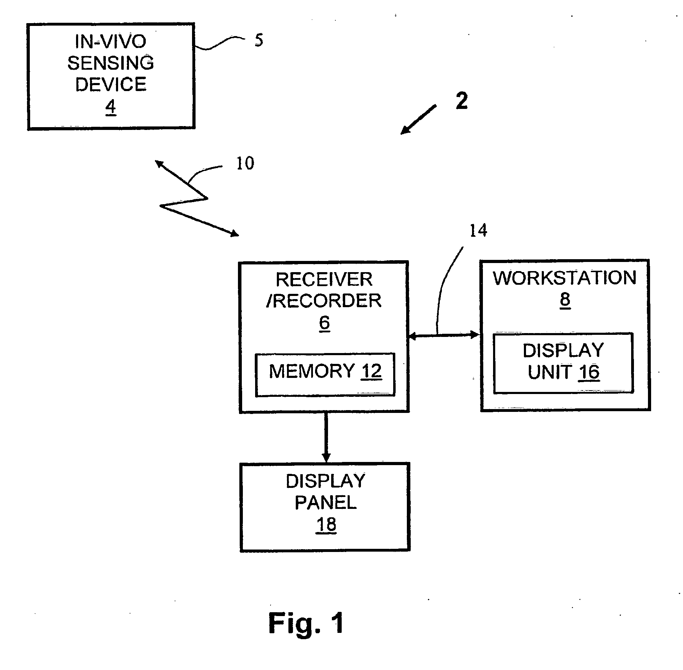

Fig. 1 is a simplified illustration of an exemplary in-vivo sensing system 2, including an in-vivo sensing device 4, a receiver/recorder 6 and a workstation 8, in accordance with some embodiments of the invention. According to some embodiments of the invention, sensing device 4 may be an oblong, oval, or spherical capsule, and may be swallowable, although other configurations are possible and are under the scope of the invention.

As illustrated in the following description, sensing device 4, contained in a housing wall 5, may be able to gather information, such as, for example, a stream of images of inner walls of body lumens while passing through inside of a patient's body, and may be able to transmit at least that information to receiver/recorder 6 outside the patient's body via a wireless or hard-wired medium 10. Receiver/recorder 6 may include a memory 12, and may be able to record information received from sensing device 4 on memory 12. Optionally, receiver/recorder 6 may include a display panel 18 which may include an LCD, TFT, CRT, OLED or other suitable panels. The display panel 18 may be integrated into receiver/recorder 6. Receiver/recorder 6 may be able to transfer the received and/or recorded information to display 18 or to workstation 8 via, for example, a wireless or hard-wired medium 14, and may be able to do so while receiving/recording information from sensing device 4.

Workstation 8 may be able to process and/or present information received from receiver/recorder 6 to an operator while sensing device 4 is still inside the patient's body, and while receiver/recorder 6 is still recording information gathered by sensing device 4. For example, workstation 8 may include a display unit 16, and may be able to display the stream of images recorded in memory 12 on display unit 16. Display unit 16 may include an LCD, TFT, CRT, OLED or other suitable medium.

By sending control signals to receiver/recorder 6 via, for example, wireless or hard-wired medium 14, workstation 8 may be able to control the way in which receiver/recorder 6 transfers recorded information to workstation 8. In view of such controls, in the example of a stream of images, receiver/recorder 6 may perform any of the following exemplary operations, although this is a non-exhaustive list: start or stop sending images to workstation 8, send the stream of images in the order received from sensing device 4 or in reverse order, start sending images to workstation 8 from a specific image in the stream, defined by, for example, a human operator of workstation 8, and the like.

Memory 12 may be fixed in or removable from receiver/recorder 6. A non-exhaustive list of examples of memory 12 includes any combination of the following semiconductor devices such as registers, latches, electrically erasable programmable read only memory devices (EEPROM), not AND (NAND) flash memory devices, not OR (NOR) flash memory devices, non-volatile random access memory devices (NVRAM), synchronous dynamic random access memory (SDRAM) devices, RAMBUS dynamic random access memory (RDRAM) devices, double data rate (DDR) memory devices, static random access memory (SRAM), universal serial bus (USB) removable memory, compact flash (CF) memory cards, personal computer memory card international association (PCMCIA) memory cards, security identity module (SIM) cards, MEMORY STICK cards, and the link; optical devices, such as compact disk read-only memory (CD ROM), compact disk recordable memory (CD-R), and the like; and magnetic devices, such as a hard disk, a floppy disk, a magnetic tape, and the like.

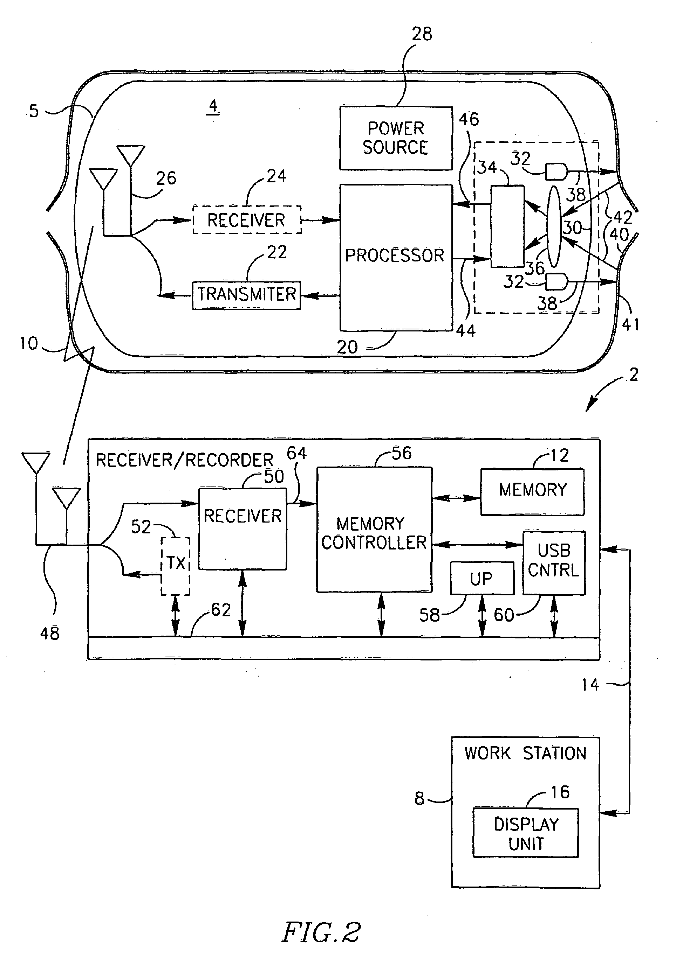

Fig. 2 is a simplified block-diagram illustration of an exemplary in-vivo sensing system 2, in accordance with some embodiments of the invention. In-vivo sensing system 2 may include a sensing device 4, a receiver/recorder 6, and a workstation 8.

According to some embodiment of the invention, sensing device 4 may be a capsule having a shell or housing 5, although other configurations are possible. Sensing device 4 may include for example an imaging system 39, a processor 20, a transmitter 22, an optional receiver 24, and at least one antenna 26. In addition, sensing device 4 may include a power source 28 to provide power to at least imaging system 39, processor 20, transmitter 22, and optional receiver 24.

Imaging system 39 may include an optical window 30, at least one illumination source 32, such as, for example, a light emitting diode (LED), an imaging sensor 34, and an optical system 36.

Illumination source 32 may produce light rays 38 that may penetrate through optical window 30 and may illuminate an inner portion 40 of a body lumen 41. A non-exhaustive list of examples of body lumen 41 includes the gastrointestinal (GI) tract, a blood vessel, the reproductive tract, or any other suitable body lumen.

Reflections 42 of light rays 38 from inner portion 40 of body lumen 41 may penetrate optical window 30 back into sensing device 4 and may be focused or directed by optical system 36 onto imaging sensor 34. Imaging sensor 34 may receive the focused reflections 42, and in response to an image capturing command 44 from processor 20, imaging sensor 34 may capture an image of inner portion 40 of body lumen 41. Processor 20 may receive the image of inner portion 40 from imaging sensor 34 over wires 46, and may control transmitter 22 to transmit the image of inner portion 40 through antenna 26 into wireless medium 10.

Sensing device 4 may passively or actively progress along an axis of body lumen 41. In time intervals that may or may not be substantially equal and may or may not be related to that progress, processor 20 may initiate capturing of an image by imaging sensor 34, and may control transmitter 22 to transmit the captured image. Consequently, a stream of images of inner portions of body lumen 41 may be transmitted from sensing device 4 into wireless medium 10.

Sensing device 4 may transmit captured images embedded in "wireless communication frames". A payload portion of a wireless communication frame may include a captured image and may include additional data, such as, for example, telemetry information and cyclic redundancy code (CRC). In addition, a wireless communication frame may include an overhead portion that may contain, for example, framing bits, synchronization bits, preamble bits, and the like.

Optional receiver 24 may be able to receive wireless messages from wireless medium 10 through antenna 26, and processor 20 may be able to capture these messages. A non-exhaustive list of examples of such messages includes activating or de-activating image capturing by sensing device 4, controlling the time intervals for capturing images, activating or de-activating transmissions from sensing device 4, or any other suitable messages.

A non-exhaustive list of examples of imaging sensor 24 includes a solid state imaging sensor, a complementary metal oxide semiconductor (CMOS) imaging sensor, a charge coupled device (CCD) imaging sensor, a linear imaging sensor, a line imaging sensor, a full frame imaging sensor, a "camera on chip" imaging sensor, or any other suitable imaging sensor.

A non-exhaustive list of examples of power source 28 includes batteries, such as, for example, silver oxide batteries, lithium batteries, capacitors, or any other suitable power source. In another embodiment of the present invention, power source 28 may not be present and the device may be powered by an external power source.

Receiver/recorder 6 may include at least one antenna 48, a receiver 50, an optional transmitter (TX) 52, a memory controller 56, a processor 58, and a communication controller, such as, for example, a universal serial bus (USB) controller 60.

Processor 58 may be able to control the operation of receiver 50, optional transmitter 52, frame synchronizer 54, memory controller 56, and USB controller 60 through a bus 62. In addition, receiver 50, optional transmitter 52, frame synchronizer 54, memory controller 56, processor 58 and USB controller 60 may be able to exchange data, such as, for example, images received from sensing device 4, or portions thereof, over bus 62.

Antenna(s) 48 may be mounted inside or outside receiver/recorder 6 and both receiver 50 and optional transmitter 52 may be coupled to antenna 48. Optional transmitter 52 may be able to transmit wireless messages to sensing device 4 through antenna 48. Receiver 50 may be able to receive transmissions, such as, for example, a stream of wireless communication frames, from sensing device 4 through antenna 48, and may output bits corresponding to the wireless communication frames on traces 64.

Receiver/recorder 6 may communicate with workstation 8 via hard-wired medium 14. For example, receiver/recorder 6 may be able to transfer recorded payloads to work station 8, and may be able to receive controls from workstation 8. Although the invention is not limited in this respect, hard-wired medium 14 may be, for example, a USB cable and may be coupled to USB controller 60 of receiver/recorder 6 and to workstation 8.

A non-exhaustive list of examples of antennae 26 and 48 includes dipole antennae, monopole antennae, multilayer ceramic antennae, Planar inverted-F antennae, loop antennae, shot antennae, dual antennae, omni-directional antennae, coil antennae or any other suitable antennas. Moreover, antenna 26 and antenna 48 may be of different types.

A non-exhaustive list of examples of processors 20 and 58 may include a central processing unit (CPU), a digital signal processor (DSP), a reduced instruction set computer (RISC), a complex instruction set computer (CISC) and the like. Moreover, processors 20 and/or 58 may each be part of an application specific integrated circuit (ASIC) or may each be a part of an application specific standard product (ASSP).

A non-exhaustive list of examples of work station 8 includes a original equipment manufacturer (OEM) dedicated work station, a desktop personal computer, a server computer, a laptop computer, a notebook computer, a hand-held computer, and the like.

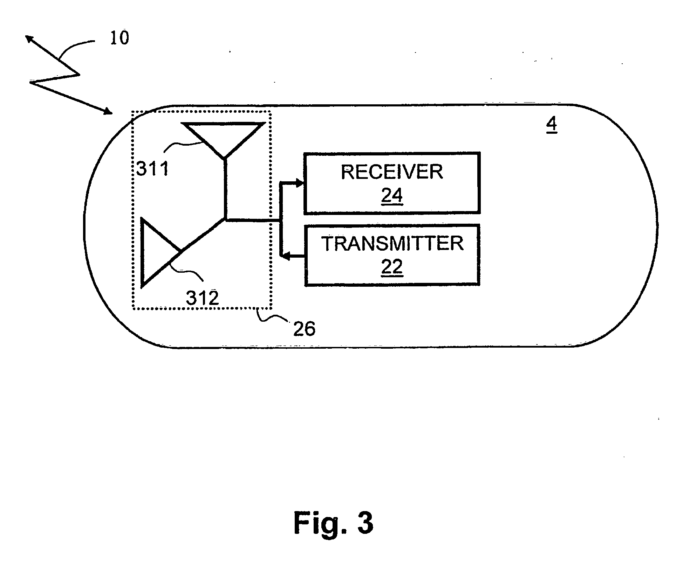

Fig. 3 is a simplified schematic illustration of a set of transmitting devices 26 (where set may include one) inside an in-vivo sensing device 4, in accordance with some exemplary embodiment of the invention. The transmitting devices may be antennas such as, for example, antennas 311 and 312, and may transmit a signal from a transmitter 22 to a wireless or hard-wired medium 10. The transmitted signal may be an image signal taken from the inside of human lumens, and may contain other telemetry data such as, for example, pH value, pressure, temperature, battery voltage, etc.

According to some embodiment of the invention, the set of antennas 26, e.g., antennas 311 and 312, may transmit same copies of a signal generated by transmitter 22. Transmitter 22 may also provide different antennas with different transmitting signals encoded with different coding scheme. For example, one antenna, such as for example antenna 311, may transmit a signal whose majority may be image signals while another antenna, such as for example antenna 312, may transmit mainly telemetry information of sensing device 4 such as, for example, battery voltage, pressure, temperature, etc.

According to some embodiment of the invention, one or more of the set of antennas 26 may work, for example, in a unidirectional mode such as receiving only or transmitting only mode. Also, one or more of the set of antennas 26 may work in a bidirectional mode of transmitting and receiving signals at the same time.

According to some embodiment of the invention, a bidirectional communication scheme by the set of antennas 26 may have transmitting and receiving signals carried by a set of carriers with a same radio frequency, or may also be carried by a set of carriers with different radio frequencies.

According to some embodiment of the invention, the set of antennas 26, e.g., antennas 311 and 312, may be oriented in different directions relative to each other such that they may be distinguished at receiver/recorder 6 (Fig. 2) by properties of received signals such as for example intensity and polarization. Consequently, the orientation of sensing device 4 may also be determined based upon combination of signals received from several different transmitting and receiving antennas. For example, antenna 311 and 312 may be placed such that they transmit signals orthogonal to each other. Other numbers of antennas may be used, such as three or more.

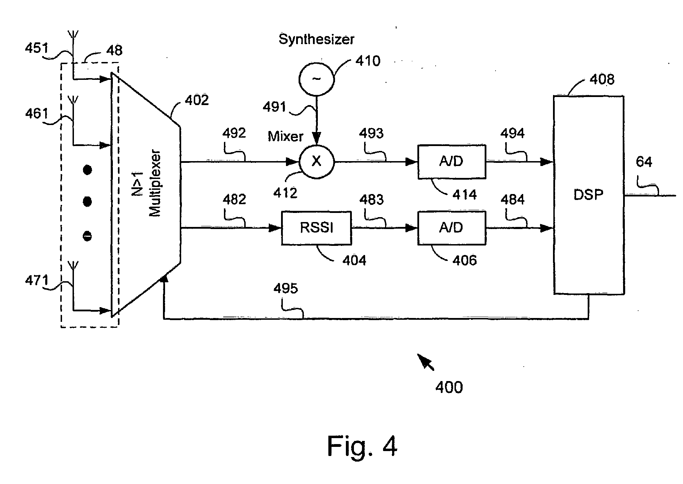

Fig. 4 is a simplified schematic block diagram illustration of a front-end receiver 400 in accordance with some exemplary embodiment of the invention. For simplicity of explanation without the loss of generality, it is assumed that transmitting devices 26 (Fig. 3) may contain only one transmitting antenna, e.g., transmitting antenna 311 (Fig. 3), and antenna 311 may transmit a signal 10 (Fig. 1) that may be received by a plurality of receiving devices 48, e.g., antennas. In other words, the plurality of antennas 48, which contains "N" antennas wherein N >1, may produce a plurality of received signals such as, e.g., signals 451, 461, and/or 471, to the input of a signal selector, e.g., multiplexer 402; other suitable signal selection devices may be used. Multiplexer 402 may further comprise antennas interface and matching circuitry and a low noise amplifier. Multiplexer 402 may produce an output signal that may be selected from the set of input signals. The selection may be based upon some pre-defined criteria such as, for example, relative signal strength. The selection of signal 492 may be controlled by a control signal 495 received from a digital signal processor (DSP) 408.

A mixer 412 may receive the selected signal 492 from multiplexer 402. Mixer 412 may also receive an oscillating frequency signal 491 from a local frequency synthesizer 410, and mix signal 491 with signal 492 from multiplexer 402 to produce a demodulated or partially demodulated signal 493. Signal 493 may be a base-band signal when the carrier frequency of signal 492 is the same, or substantially the same, as the oscillating frequency of signal 491 from synthesizer 410. Signal 493 may also be a signal having a carrier of an intermediate frequency (IF), being the difference between carrier frequency of signal 492 and frequency of synthesizer 410, which is downward converted from the original radio frequency (RF).

The mixer 412 may apply the base-band signal 493, or the partially demodulated signal 493 with an IF carrier to an analog-to-digital (A/D) converter 414, and signal 493 may be converted into a digital signal 494. A digital signal processor (DSP) 408 may process digital signal 494 received from A/D converter 414, and provide an output signal to traces 64 (Fig. 2).

Multiplexer 402 may periodically tap the powers of input signals such as, e.g., signals 451, 461, and/or 471 and output as signal 482 comprising tapped signal powers. A relative signal strength indicator (RSSI) unit 404 may measure the signal strength of input signal 482, select an input signal that has the strongest power, and send a signal strength indication signal 483 to an analog-to-digital (A/D) converter 406. A/D converter 406 may then convert signal 483 into a digital signal 484 to apply to DSP unit 408. DSP unit 408 may switch the selection of output of multiplexer 402, based on signal 484, to an input that may be selected by the RSSI unit 404. DSP 408 may provide the control of multiplexer 402 through a control signal 495.

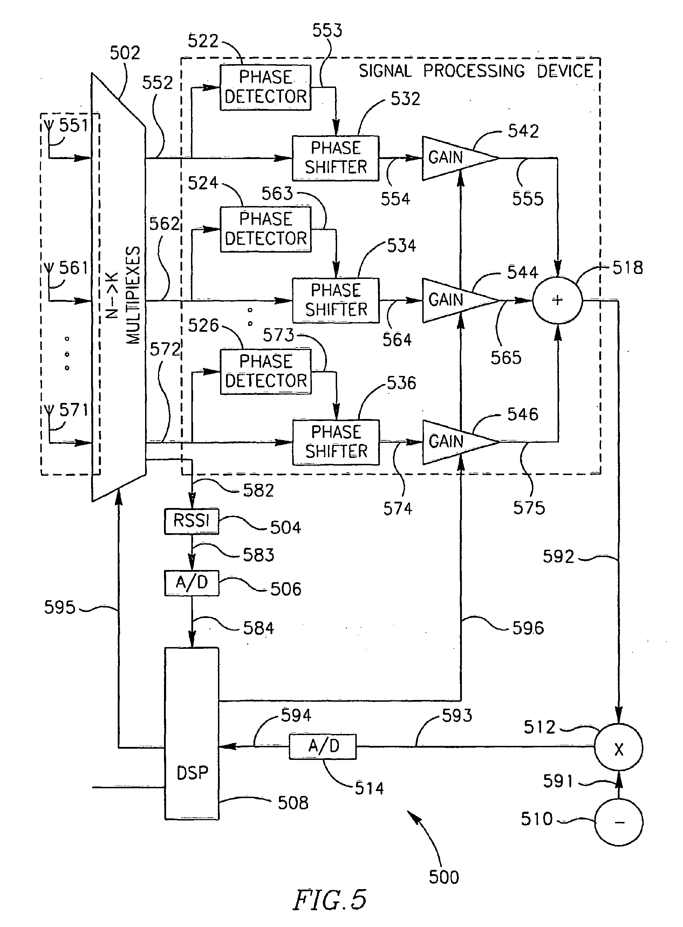

Fig. 5 is a simplified schematic block diagram illustration of a front-end receiver 500, in accordance with some exemplary embodiment of the invention. A plurality of receiving devices 48, e.g., "N" antennas wherein N>1, may detect a signal transmitted for example, by one of transmitting devices 26 such as for example antenna 311 (Fig. 3). In other words, the plurality of antennas 48 may produce N input signals such as, e.g., signals 551, 561, and 571, to a signal selector, e.g., multiplexer 502.

Multiplexer 502 may have N input ports and "k" output ports. Output signals from the k output ports, wherein 1<k<N and preferably equals two but need not be, may be selected from the N input signals, and the selection may be controlled by a control signal 595 from a digital signal processor (DSP) 508. The selection may be based upon some pre-defined criteria such as, for example, relative signal strength among the N input signals.

It is appreciated in the following that the number of output signals "k" from signal selector, e.g., multiplexer 502, may be conveniently illustrated by three signals without the loss of generality. The number of output signals k may preferably be two, or other numbers.

The k output signals from multiplexer 502 such as, for example, signals 552, 562, and 572, may be input signals to a set of phase shifters such as, for example, 532, 534, and 536. At the same time, at least a portion of signals 552, 562, and 572 may be tapped off to provide inputs to a set of phase detectors such as, for example, detectors 522, 524, and 526, respective to the set of phase shifters 532, 534, and 536.

According to some exemplary embodiments of the invention, phase detector 522, for example, may detect and measure phase information of input signal 552, and the measured phase information may be compared with a pre-defined reference phase. The same reference phase may also be used in other phase detectors such as, for example, detectors 524 and 526. In other words, phase information of signal 552 may effectively be compared with other output signals from multiplexer 502 such as, for example, signals 562 and 572 as is illustrated here. In one embodiment of the invention, phase information of one signal, e.g., signal 552, may be compared directly with other signals, e.g., those of signals 562 and 572.

Based upon the difference between the phase of signal 552 and the reference phase, phase detector 522 may output a control signal 553, and the control signal 553 may be applied to a phase shifter 532. Phase shifter 532, working together with other phase shifters, e.g., 534 and 536 and controlled by signals 563 and 573 respectively, may provide a delay time adjustment to the input signal 552, such that an output signal 554 from phase shifter 532 may be in substantially the same phase, or in-phase, with signals from other phase shifters, e.g., signals 564 and 574 from phase shifters 534 and 536. In other words, signals 554, 564, and 574 may be in-phase after phase shifters 532, 534, and 536.

In situations where signals 554, 564, and 574 may already have substantially the same strength without further amplitude adjustment, and may therefore be added together by a combiner 516 to produce a combined signal 592 with enhanced signal-to-noise (SNR) ratio.

Alternatively, the strength of the set of signals 554, 564, and 574 may be adjusted by a set of RF amplifiers such as, for example, amplifiers 542, 544, and 546, to have substantially the same amplitudes or signal strength. The k signals, for example, signals 555, 565, and 575, coming out of the set of amplifiers, e.g., 542, 544, and 546, are added together by a combiner 516, to provide a combined signal 592 with enhanced signal-to-noise ratio (SNR) Normally, when two signals, e.g., signals 555 and 565, with subsequently the same signal amplitude are added together, an enhancement of SNR of up to 3-dB may be achieved since the signal amplitude may be twice as bigger, corresponding to a factor of four increase in signal power, compared with the increase of noise power by a factor of two. A k of number of signals larger than two may further increase the SNR but may come at the expense of increased hardware complexity.

The phase detectors, e.g., detector 522, 524, and 526, phase shifters, e.g., phase shifter 532, 5.34, and 536, amplifiers, e.g., amplifier 542, 544, and 546, and combiner 516 may be collectively referred to as a signal processing device. The signal processing device receives multiple inputs from the outputs of the multiplexer 502, and provides a single output signal 592.

A mixer 512 may receive the combined signal 592 from the combiner 516. Mixer 512 may also receive an oscillating frequency signal 591 from a local frequency synthesizer 510, and mix signal 591 with signal 592 from combiner 516 to produce a demodulated signal 593. Signal 593 may be a base-band signal, when the carrier frequency of signal 592 is the same, or substantially the same, as the oscillating frequency of signal 591 from synthesizer 510, and/or may be a signal having a carrier at an intermediate frequency (IF), which is the difference between carrier frequency of signal 592 and frequency of signal 591, that may be downward converted from the original radio frequency (RF).

As is described above, the selection of the k output signals by multiplexer 502 is controlled by signal 595. In addition, gains of the set of amplifiers 542, 544, and 546 may also be controlled by a control signal 596, wherein both signal 595 and 596 are produced and controlled by DSP unit 508.

Digital signal processor 508 may provide control signal 595 based upon a sampled signal 582 provided by multiplexer 502. Signal 582 may be a portion of one of the input signals, e.g., signals 551, 561, or 571. In other words, multiplexer 502 may periodically tap into one of the input signals 551, 561, or 571, and provide an output signal 582. A relative signal strength indicator (RSSI) unit 504 may measure the signal strength of its input 582, across sampled input signals, and output a signal strength indication signal 583 to an analog-to-digital (A/D) converter 506, where signal 583 may be converted into digital signal 584 and applied to DSP unit 508. Based on signal 584 that provides signal strength information across N input signals, e.g., signals 551, 561, and 571, DSP unit 508 may provide a control signal 595 to control the selection of input signals by multiplexer 502 so that k signals with the relative strong signal strength may be selected.

Digital signal processor 508 may also provide control signal 596 based upon quality of detected base-band signal 594 provided by A/D converter 514 as digital signal. DSP 508 may adjust the amount of gains of individual amplifiers, e.g., amplifiers 542, 544, and 546, and monitor the quality of output signal from A/D converter 514, such as, for example, signal strength level, noise power, or SNR. As is described above, DSP 508 may control the gains of amplifiers such that output signals from amplifiers, e.g., signals 555, 565, and/or 575, may have substantially the same signal strength.

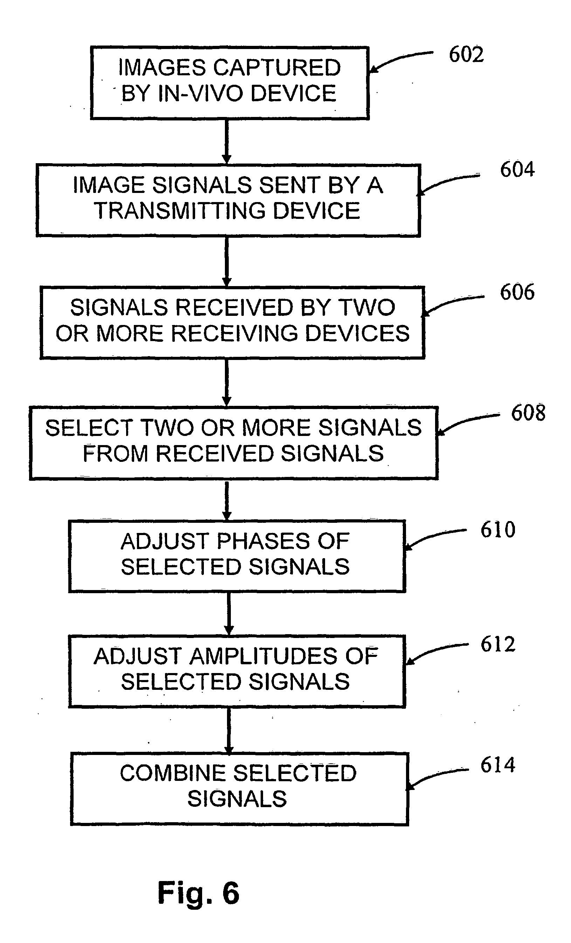

Fig. 6 is a simplified block diagram illustration of a method according to an embodiment of the invention. According to one embodiment a method as described, for example, in Fig. 5 and Fig. 6, may be used for reproducing a transmitted signal from an in-vivo sensing device. An in-vivo sensing device may first capture image signals from the inside of human lumens (block 602). The captured image signals may then be sent by one or more transmitting devices, e.g., antennas, to the outside of the human body (block 604). A plurality of receiving devices, e.g., antennas, may subsequently produce a plurality of received signals transmitted by the in-vivo sensing device (block 606). Two or more of the received signals, for example, signals with the strongest signal strength may be selected (block 608). The selected two or more signals may be adjusted to be in-phase with each other (block 610), and to have substantially the same amplitudes or signal strengths (block 612). Other parameters of the signal may be adjusted. The selected two or more signals, after being adjusted, for example, for phase and amplitude, may be combined together to produce an output signal that may represent the transmitted signal (614). Alternatively, the selected two or more signals may be combined together (614) directly after being adjusted to be in-phase or substantially in-phase (610) when signal strengths of the signals are relatively close to each other.

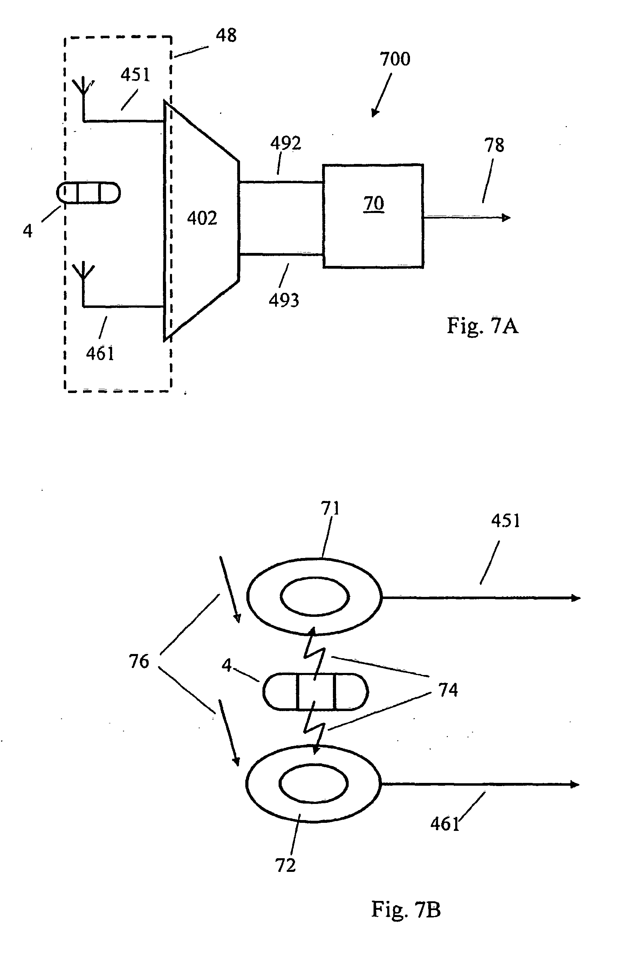

Figs. 7A and 7B are simplified schematic block diagram illustration and schematic physical illustration, respectively, of a part of a front-end receiver 700 in accordance with some exemplary embodiments of the invention. Front-end receiver 700 may comprise an antenna set 48 comprising at least two antennas 451, 461, a multiplexer 402 that may control signals from which of antennas 451, 461 are passed on for further processing, and a processor 70. Processor 70 may further comprise means for amplitude correction and control, means for phase correction and control, two-signal subtractor and impedance matching and output gain control.

Antennas 451, 461 with respective receiving ends 71, 72 are selected by multiplexer 402, so that in-vivo sensing device 4 is situated substantially between the two selected antennas. Transmissions 74 from sensing device 4 are received in receiving end 71 of antenna 451 so that, in the illustrated example of Fig. 7B, the magnetic flux flows from bottom to top of receiving end 71 while with receiving end 72 of antenna 461 that flux flows from top to bottom of the receiving end. On the other hand, an external transmission 76, such as transmission from a proximate transmitting device or that of an electromagnetic noise, is received by antennas 451, 461 its flux passes through receiving ends 71, 72 in the same direction, i.e. from top to bottom in the illustrated example of Fig. 7B. As a result the portions of the signal in lines 492, 493 representing the capsule signal are added to each other and thus the signal received from sensing device 4 is increased. On the other hand, the portions of signals received from the external source are substantially mutually cancelled. Beside the cancellation of the interference from the external source the SNR (signal to noise ratio) of the capsule signal is improved by up to 3 dB. The strength and quality of the signal received from sensing device 4 may further be improved by the adjustment of the phase and amplitude of the signals from the two antennas 451, 452. This may be carried out by processor 70.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the scope of the invention.

## Claims

1. An in-vivo communication system comprising:
an in-vivo sensing device comprising a transmitting device; and
a reception system comprising:
a plurality of receiving devices;
a signal selector connected to said plurality of receiving devices; and
a signal processing device connected to said signal selector,
wherein said sensing device is able to communicate with said reception system through said transmitting device and said plurality of receiving devices, and said signal selector is able to select two or more signals from a plurality of received signals provided by said plurality of receiving devices, and output said two or more signals to said signal processing device;
**characterised in that** said signal processing device is able to adjust said selected two or more signals to be substantially in-phase.

2. The in-vivo communication system of claim 1, wherein said signal selector measures a relative signal strength of said plurality of received signals and selects said two or more signals based upon an order of said relative signal strength.

3. The in-vivo communication system of claim 1, wherein said signal processing device is able to combine said phase adjusted two or more signals substantially in-phase.

4. The in-vivo communication system of claim 1, wherein said signal processing device is able to adjust said phase adjusted two or more signals to have substantially same amplitudes.

5. The in-vivo communication system of claim 4, wherein said signal processing device is able to combine said phase and amplitude adjusted two or more signals substantially in-phase and in substantially same amplitudes.

6. The in-vivo communication system of claim 1, wherein said in-vivo sensing device is a swallowable capsule.

7. The in-vivo communication system of claim 1, wherein said transmitted signal comprises an image signal.

8. The in-vivo communication system of claim 1, wherein said transmitting device comprises a transmitting antenna.

9. The in-vivo communication system of claim 1, wherein said plurality of receiving devices comprises two or more receiving antennas.

10. The in-vivo communication system of claim 1, wherein said signal selector selects two of said plurality of receiving devices being situated substantially on opposite sides of said in-vivo sensing device.

11. The in-vivo communication system of claim 10, wherein the signals received from said two selected receiving devices are in-phase and amplitude-adjusted.

12. A method of reproducing a transmitted signal from an in-vivo sensing device, the method comprising:
receiving a plurality of signals at a plurality of receiving devices;
selecting two or more signals from said plurality of received signals;
constructing said transmitted signal from said selected two or more signals;
detecting phases of said selected two or more signals; and
adjusting said phases of said selected two or more signals to be substantially in-phase.

13. The method of claim 12, wherein said adjusting said phases comprises changing delay times to said selected two or more signals.

14. The method of claim 12, further comprising:
combining said phase-adjusted two or more signals to reproduce said transmitted signal.

15. The method of claim 12, further comprising:
measuring amplitudes of said phase adjusted two or more signals; and
adjusting said amplitudes of said phase adjusted two or more signals to have substantially the same amplitudes.

16. The method of claim 15, further comprising:
combining said phase and amplitude adjusted two or more signals to reproduce said transmitted signal.

17. The method of claim 12, wherein said selecting comprises selecting two or more signals from said plurality of received signals by an order of relative signal strength.

18. The in-vivo communication system of claim 1, wherein said signal selector comprises a multiplexer.

19. The in-vivo communication system of claim 18, wherein said in-vivo sensing device comprises an imager.

## Patentansprüche

1. In-vivo Kommunikationssystem, umfassend:
eine in-vivo Sensorvorrichtung mit einer Sendevorrichtung; und
einem Empfangssystem, umfassend:
- eine Vielzahl von Empfangsvorrichtungen;
- eine Signalauswahleinheit, verbunden mit der Vielzahl von Empfangsvorrichtungen; und
- eine Signalverarbeitungsvorrichtung, verbunden mit der Signalauswahleinheit,
wobei die Sensorvorrichtung in der Lage ist, mit dem Empfangssystem über die Sendevorrichtung und die Vielzahl von Empfangsvorrichtungen zu kommunizieren, und die Signalauswahleinheit in der Lage ist, zwei oder mehr Signale aus einer Vielzahl von durch die Vielzahl von Empfangsvorrichtungen bereitgestellten, empfangenen Signalen auszuwählen, und die zwei oder mehr Signale an die Signalverarbeitungsvorrichtung auszugeben;
**dadurch gekennzeichnet,**
**dass** die Signalverarbeitungsvorrichtung in der Lage ist, die ausgewählten zwei oder mehr Signale anzupassen, um im Wesentlichen in Phase zu sein.

2. In-vivo Kommunikationssystem nach Anspruch 1, wobei die Signalauswahleinheit eine relative Signalstärke der Vielzahl von empfangenen Signalen misst und die zwei oder mehr Signale auf Grundlage einer Rangfolge der relativen Signalstärke auswählt.

3. In-vivo Kommunikationssystem nach Anspruch 1, wobei die Signalverarbeitungsvorrichtung in der Lage ist, die Phasen-angepassten zwei oder mehr Signale im Wesentlichen in Phase zu kombinieren.

4. In-vivo Kommunikationssystem nach Anspruch 1, wobei die Signalverarbeitungsvorrichtung in der Lage ist, die Phasen-angepassten zwei oder mehr Signale anzupassen, um im Wesentlichen gleiche Amplituden aufzuweisen.

5. In-vivo Kommunikationssystem nach Anspruch 4, wobei die Signalverarbeitungsvorrichtung in der Lage ist, die Phasen- und Amplituden-angepassten zwei oder mehr Signale im Wesentlichen in Phase und im Wesentlichen mit gleichen Amplituden zu kombinieren.

6. In-vivo Kommunikationssystem nach Anspruch 1, wobei die in-vivo Sensorvorrichtung eine schluckbare Kapsel ist.

7. In-vivo Kommunikationssystem nach Anspruch 1, wobei das gesendete Signal ein Bildsignal umfasst.

8. In-vivo Kommunikationssystem nach Anspruch 1, wobei die Sendevorrichtung eine Sendeantenne umfasst.

9. In-vivo Kommunikationssystem nach Anspruch 1, wobei die Vielzahl von Empfangsvorrichtungen zwei oder mehr Empfangsantennen umfasst.

10. In-vivo Kommunikationssystem nach Anspruch 1, wobei die Signalauswahleinheit zwei aus der Vielzahl von Empfangsvorrichtungen auswählt, die sich im Wesentlichen auf gegenüberliegenden Seiten der in-vivo Sensorvorrichtung befinden.

11. In-vivo Kommunikationssystem nach Anspruch 10, wobei die von den zwei ausgewählten Empfangsvorrichtungen empfangenen Signale in Phase und Amplituden-angepasst sind.

12. Verfahren zum Reproduzieren eines gesendeten Signals von einer in-vivo Sensorvorrichtung, wobei das Verfahren umfasst:
Empfangen einer Vielzahl von Signalen bei einer Vielzahl von Empfangsvorrichtungen;
Auswählen von zwei oder mehr Signalen aus der Vielzahl von empfangen Signalen;
Konstruieren des gesendeten Signals aus den ausgewählten zwei oder mehr Signalen;
Erfassen der Phasen der ausgewählten zwei oder mehr Signale; und
Anpassen der Phasen der ausgewählten zwei oder mehr Signale, um im Wesentlichen in Phase zu sein.

13. Verfahren nach Anspruch 12, wobei das Anpassen der Phasen ein Verändern von Verzögerungszeiten bei den zwei oder mehr ausgewählten Signalen umfasst.

14. Verfahren nach Anspruch 12, ferner umfassend:
Kombinieren der Phasen-angepassten zwei oder mehr Signale, um das gesendete Signal zu reproduzieren.

15. Verfahren nach Anspruch 12, ferner umfassend:
Messen von Amplituden der Phasen-angepassten zwei oder mehr Signale; und
Anpassen der Amplituden der Phasen-angepassten zwei oder mehr Signale, um im Wesentlichen die gleichen Amplituden aufzuweisen.

16. Verfahren nach Anspruch 15, ferner umfassend:
Kombinieren der Phasen- und Amplituden-angepassten zwei oder mehr Signale zum Reproduzieren des gesendeten Signals.

17. Verfahren nach Anspruch 12, wobei das Auswählen ein Auswählen von zwei oder mehr Signalen aus der Vielzahl von empfangenen Signalen nach einer Rangfolge der relativen Signalstärken umfasst.

18. In-vivo Kommunikationssystem nach Anspruch 1, wobei die Signalauswahleinheit einen Multiplexer umfasst.

19. In-vivo Kommunikationssystem nach Anspruch 18, wobei die in-vivo Sensorvorrichtung einen Bildgeber umfasst.

## Revendications

1. Système de communication in-vivo comprenant:
un dispositif de détection in-vivo comprenant un dispositif de transmission; et
un système de réception comprenant:
une pluralité de dispositifs de réception;
un sélecteur de signaux connecté à ladite pluralité de dispositifs de réception; et
un dispositif de traitement des signaux connecté audit sélecteur de signaux,
dans lequel ledit dispositif de détection est en mesure de communiquer avec ledit système de réception à travers ledit dispositif de transmission et ladite pluralité de dispositifs de réception, et
ledit sélecteur de signaux est en mesure de sélectionner deux signaux ou plus à partir d'une pluralité de signaux reçus procurés par ladite pluralité de dispositifs de réception, et délivrer en sortie deux signaux ou plus audit dispositif de traitement des signaux;
**caractérisé en ce que** ledit dispositif de traitement des signaux est en mesure d'ajuster lesdits deux signaux sélectionnés ou plus pour qu'ils soient essentiellement en phase.

2. Système de communication in-vivo de la revendication 1, dans lequel ledit sélecteur de signaux mesure une intensité relative de signal de ladite pluralité de signaux reçus et sélectionne lesdits deux signaux ou plus sur la base d'un ordre de ladite intensité relative de signal.

3. Système de communication in-vivo de la revendication 1, dans lequel ledit dispositif de traitement des signaux est en mesure de combiner essentiellement en phase lesdits deux signaux à phases ajustées ou plus.

4. Système de communication in-vivo de la revendication 1, dans lequel ledit dispositif de traitement des signaux est en mesure d'ajuster lesdits deux signaux à phases ajustées ou plus pour qu'ils aient essentiellement les mêmes amplitudes.

5. Système de communication in-vivo de la revendication 4, dans lequel ledit dispositif de traitement des signaux est en mesure de combiner essentiellement en phase et essentiellement aux mêmes amplitudes lesdits deux signaux à phases et amplitudes ajustées ou plus.

6. Système de communication in-vivo de la revendication 1, dans lequel ledit dispositif de détection in-vivo est une capsule avalable.

7. Système de communication in-vivo de la revendication 1, dans lequel ledit signal transmis comprend un signal d'image.

8. Système de communication in-vivo de la revendication 1, dans lequel ledit dispositif de transmission comprend une antenne de transmission.

9. Système de communication in-vivo de la revendication 1, dans lequel ladite pluralité de dispositifs de réception comprend deux antennes de réception ou plus.

10. Système de communication in-vivo de la revendication 1, dans lequel ledit sélecteur de signaux sélectionne deux parmi ladite pluralité de dispositifs de réception qui sont situés essentiellement sur des côtés opposés dudit dispositif de détection in-vivo.

11. Système de communication in-vivo de la revendication 10, dans lequel les signaux reçus à partir desdits deux dispositifs de réception sélectionnés sont ajustés en phase et en amplitude.

12. Procédé pour reproduire un signal transmis à partir d'un dispositif de détection in-vivo, le procédé comprenant:
la réception d'une pluralité de signaux à une pluralité de dispositifs de réception;
la sélection de deux signaux ou plus à partir de ladite pluralité de signaux reçus;
la construction dudit signal transmis à partir desdits deux signaux sélectionnés ou plus;
la détection des phases desdits deux signaux sélectionnés ou plus; et
l'ajustement desdites phases desdits deux signaux sélectionnés ou plus pour qu'ils soient essentiellement en phase.

13. Procédé de la revendication 12, dans lequel ledit ajustement desdites phases comprend le changement de temps de retards auxdits deux signaux sélectionnés ou plus.

14. Procédé de la revendication 12, comprenant en plus:
la combinaison desdits deux signaux ou plus à phases ajustées pour reproduire ledit signal transmis.

15. Procédé de la revendication 12, comprenant en plus:
la mesure des amplitudes desdits deux signaux à phases ajustées ou plus; et
l'ajustement desdites amplitudes desdits deux signaux à phases ajustées ou plus pour qu'ils aient essentiellement les mêmes amplitudes.

16. Procédé de la revendication 15, comprenant en plus:
la combinaison desdits deux signaux à phases et amplitudes ajutées ou plus pour reproduire ledit signal transmis.

17. Procédé de la revendication 12, dans lequel ladite sélection comprend la sélection de deux signaux ou plus à partir de ladite pluralité de signaux reçus par un ordre d'intensité relative de signal.

18. Système de communication in-vivo de la revendication 1, dans lequel ledit sélecteur de signaux comprend un multiplexeur.

19. Système de communication in-vivo de la revendication 18, dans lequel ledit dispositif de détection in-vivo comprend un imageur.
